Europäisches Patentamt

European Patent Office

Office européen des brevets

(11) Veröffentlichungsnummer: **0 285 949 B1**

(12) **EUROPÄISCHE PATENTSCHRIFT**

(45) Veröffentlichungstag der Patentschrift: **24.03.93**

(21) Anmeldenummer: **88104953.0**

(22) Anmeldetag: **28.03.88**

(51) Int. Cl.⁵: **C12N 15/75**, C12N 1/21, C12P 21/00, C12N 9/04, C12N 9/10, //(C12N1/21, C12R1:11)

(54) **Genetische Kontrolleinheit und Klonierungs- und Expressionssystem.**

(30) Priorität: **08.04.87 DE 3711883**

(43) Veröffentlichungstag der Anmeldung:
**12.10.88 Patentblatt 88/41**

(45) Bekanntmachung des Hinweises auf die
Patenterteilung:
**24.03.93 Patentblatt 93/12**

(84) Benannte Vertragsstaaten:
**CH DE ES FR GB IT LI NL SE**

(56) Entgegenhaltungen:
**EP-A- 0 133 756**
**EP-A- 0 146 901**
**EP-A- 0 227 260**
**GB-A- 2 142 336**

**JOURNAL OF BACTERIOLOGY, Band 169, Nr. 5, Mai 1987, Seiten 2165-2170, American Society for Microbiology, Baltimore, US; O.A. CARMI et al.: "Use of bacterial luciferase to establish a promotor probe vehicle capable of nondestructive real-time analysis of gene expression in bacillus spp."**

(73) Patentinhaber: **MERCK PATENT GESELL-SCHAFT MIT BESCHRÄNKTER HAFTUNG**
**Frankfurter Strasse 250 Postfach 4119**
**W-6100 Darmstadt(DE)**

(72) Erfinder: **Ebeling, Wolfgang, Dr.**
**Am Hintergraben 9**
**W-6101 Bickenbach(DE)**
Erfinder: **Heilmann, Hans-Jürgen, Dr.**
**Schöneweibergasse 91**
**W-6103 Griesheim(DE)**
Erfinder: **Meinhardt, Friedhelm, Dr.**
**Grosse Erckenschwickerstrasse 95**
**W-4353 Oer-Erckenschick(DE)**

**Beschreibung**

Die Erfindung betrifft die Bereitstellung einer genetischen Kontrolleinheit, welche mindestens einen Promotor-Bereich, gekennzeichnet durch die DNA-Sequenz I:

```
AAG CTT CTC ATT ATC ACA AAA TGG GAA TTG TTA TTT
CAA CGC TTG GCG GTA TCT TCT TAT TAG GAG AAA AGA
AAA CGA AGC GTC AGC TTA TTT CGA TTA CGA TTG GTA
TTG TGC TCA TCA TTT TAG GTG GCG TAT TCC TCG GAA
TGG CTA AAA GTT AAC GTT TAC GTA AAA GTT AAT GAT
AGT ATG TAT GAA TAA ATG AAA ACA CTA GGA GGA TTT
TAT T
```

enthält.

Die Erfindung betrifft zudem die Bereitstellung einer Terminator-Region, die durch die DNA-Sequenz II:

```
TTT TTA ATC GAT GTA GAA AAA AGA GAC ATG GCA AAA
AGC GCC ATG TCT CTT TTT TCT ATA CCT AAG CGA ATG
ACA CCC CCC TTT CGG GGT TGG TGG CAT TCG CCT AGG
TAA AAG CTT
```

repräsentiert wird.

Die Erfindung betrifft weiterhin Klonierungs- und Expressionsvektoren zur Verwendung bei der Herstellung von Proteinen sowie mit solchen Vektoren transformierte Wirtsorganismen.

Außerdem betrfft die Erfindung ein Bakterien-Klonierungs-und Expressions-System, welches einen transformierbaren, vorzugsweise mikrobiellen Wirtsorganismus, die oben spezifizierte, vorzugsweise aus der Art Bacillus megaterium stammenden Kontrolleinheit sowie ein homologes oder heterologes Strukturgen enthält. Dabei können anstelle der erfindungsgemäßen Terminator-Region gemäß DNA-Sequenz II gegebenenfalls auch andere wirksame Terminator-Bereiche bzw. DNA-Sequenzen Verwendung finden.

Schließlich betrifft die Erfindung ein gentechnisches Verfahren zur Herstellung von arteigenen oder artfremden Proteinen unter Verwendung des genannten Klonierungs-und Expressionssystems.

Durch die Einführung der Rekombinations-DNA-Technik ist der Biotechnologie ein Verfahren zur Gewinnung von Proteinen zur Verfügung gestellt worden, welches die Möglichkeit bietet, arteigene (homologe) Proteine oder durch entsprechende Manipulationen denkbare heterologe Proteine beispielsweise durch Genamplifikation in einem Wirtsorganismus überproduzieren zu lassen. Hierfür werden sehr oft E. coli-Bakterien als Wirtsorganismen eingesetzt, die nicht nur in einem gewissen Maß artfremde Transkriptions-und Translationssignale akzeptieren, sondern darüberhinaus selbst starke und gut regulierbare Promotoren besitzen und somit auch Fremdgene mit zum Teil befriedigenden Umsatzraten zu exprimieren vermögen. Andere Mikroorganismen (z.B. Bacillus subtilis zur $\alpha$-Amylase-Gewinnung gemäß der DE-OS-31 52 001) können als Wirtsorganismus noch nicht in dem Maße wie E. coli eingesetzt werden. Gründe hierfür sind unter anderem, daß in diesen Organismen, wie z.B. in Bacillus subtilis, häufig strukturelle Instabilitäten der verwendeten Vektoren in den transformierten Zellen aufgrund z.B. von Deletionen, Rearrangements usw. beobachtet werden. Weiterhin besitzen Bacillus-Arten im Gegensatz zu E. coli im wesentlichen nur spezifische, arteigene Erkennungs- und Regulations-Sequenzen, d.h. Fremdgene können nicht oder nur schlecht exprimiert werden. Zudem sind nur wenige starke und regulierbare Promotoren bzw. Kontrolleinheiten für Bacillus bekannt.

Andererseits ist der Einsatz von Gram-positiven Bakterien auch für gentechnologische Anwendungen vorteilhaft und somit wünschenswert. So sind Bacillus-Arten, wie z.B. Bacillus subtilis oder Bacillus megaterium im Gegensatz zu einer Reihe von E. coli-Stämmen apathogen. Überdies sind sie im Gegensatz zu E. coli-Bakterien, die als Fäkalien-Anzeiger gelten, ohne Probleme einsetzbar auf Gebieten, die strengen

EP 0 285 949 B1

Hygiene-Vorschriften unterliegen, wie z.B. in der Lebensmittelbranche oder auf dem Trinkwassersektor. Weiterhin sind Fermentationstechnik und Produktpalette für Bacillus-Arten gut bekannt. Molekularbiologisch besitzen sie gegenüber gram-negativen Bakterien den Vorteil, Proteine in löslicher Form auszuscheiden. Sie sind daher besonders gut zur Konstruktion von Exkretionsvektoren geeignet.

Es besteht also aus mehrfachen Gründen ein Bedürfnis, weitere praktikable und produktive, vorzugsweise Nicht-E. coli-Systeme zu entwickeln, um der wachsenden wirtschaftlichen Bedeutung der Gentechnologie Rechnung zu tragen und eine immer vielfältigere Palette von wertvollen Substanzen, insbesondere Proteinen, für die verschiedenen Anwendungsbereiche zur Verfügung zu stellen.

Der Erfindung liegt somit die Aufgabe zugrunde, ein neues Klonierungs- und Expressionssystem mit einer starken Kontrolleinheit und stabilen Vektoren bereitzustellen, welches in der Lage ist, endogene und exogene Proteine in hoher Ausbeute und guter Qualität zu synthetisieren.

Diese Aufgabe wurde, wie nachfolgend aufgeführt, gelöst.

Überraschend wurde gefunden, daß durch Anlegen einer Genbank von Bacillus megaterium in einem Phagenvektor, vorzugsweise λ-EMBL3, sukzessive Verkleinerung eines DNA-Bereiches, der für das Enzym Glucose-Dehydrogenase codiert, und durch Transformation eines Zwischenwirtes, vorzugsweise E. coli, eine DNA-Sequenz erhalten wird, die (neben dem Stukturgen) eine Promotor- und eine Terminator-Region gemäß der DNA-Sequenzen I und II enthält. Diese bewirken nach Insertion in geeignete Vektoren und Klonierung in einem vorzugsweise mikrobiellen Wirtsorganismus, z.B. der Gattung Bacillus, die Expression von verschiedenen unter ihrer Kontrolle stehenden Strukturgenen in überraschend hohen Ausbeuten und hervorragender Qualität.

Erfindungsgemäß ist der Terminus "... unter ihrer Kontrolle stehende Strukturgene ..." so zu verstehen, daß Kontrolleinheit und Strukturgene entweder direkt miteinander verbunden sind, oder aber daß zwischen Kontrolleinheit und Strukturgen nicht codierende oder anderweitig codierende DNA-Abschnitte inseriert sein können.

Als Ausgangs-Spezies dienen Mikroorganismen der Art Bacillus megaterium sowie deren Mutanten oder Varianten, welche die entsprechende genetische Information besitzen. Stämme dieser Art sind bekannt und bei hierfür autorisierten Hinterlegungsstellen hinterlegt, z.B. DSM 321, DSM 322, DSM 333 oder DSM 337.

Als Wirtsorganismen, die mit den erfindungsgemäßen DNA-Sequenzen I und II transformiert werden können, kommen in erster Linie Mikroorganismen, aber auch pflanzliche, tierische oder menschliche Zellen in Betracht vorzugsweise werden aber Bakterien wie E. coli oder Bacillus-Arten, insbesondere Bacillus megaterium eingesetzt.

Als Strukturgene eignen sich sowohl homologe Gene, also solche, die der Wirt von Natur aus enthält, als auch Fremdgene. So lassen sich die Glucose-Dehydrogenase-Gene (I) und (II) (Erläuterung hierzu siehe unten) und das Chloramphenicol-Acetyltransferase-Gen unter die Kontrolle der erfindungsgemäßen Kontrolleinheit stellen und exprimieren. Aber auch andere Gene, wie z.B. das Mutarotase-Gen, das α-Amylase-Gen, das ß-Glucosidase-Gen, das β-Galactosidase-Gen, Transaminase-, Lipase- oder Protease-Gene sind prinzipiell exprimierbar.

Die Effektivität der erfindungsgemäßen Kontrolleinheit, inbesondere des Promotors im homologen Wirt ist beträchtlich. So liegt beispielsweise die Aktivität der exprimierten Glucose-Dehydrogenase-(I) im Wirt der Art Bacillus megaterium zwischen 20 und 65 Units/ml Kultur, vorzugsweise zwischen 35 und 50 Units/ml. Die Expression unter Kontrolle des erfindungsgemäßen Promotors von Glucose-Dehydrogenase-(II) liegt unter den gleichen Bedingungen zwischen 10 und 35, vorzugsweise zwischen 15 und 25 Units/ml, ohne den erfindungsgemäßen Promotor aber nur zwischen 0,05 und 0,2 Units/ml Kultur für beide Gene. Die entsprechenden Werte für Chloramphenicol-Acetyltransferase (unter Kontrolle des erfindungsgemäßen Promotors) liegen zwischen 500 und 1500, vorzugsweise zwischen 600 und 1300 mUnits/ml Kultur. Dies hat zur Folge, daß beispielsweise Bacillus megaterium-Stämme, die ein CAT-Gen unter der Kontrolle der erfindungsgemäßen Promotor-Sequenz I tragen, etwa 30 bis 50mal resistenter gegen das entsprechende Antibiotikum sind als nicht transformierte Stämme. Die von der erfindungsgemäßen Kontrolleinheit bewirkten hohen Expressionswerte werden in E. coli bislang nur unter Verwendung sehr starker E. coli-Promotoren, wie beispielsweise des bekannten λ-P_L-Promotors, erreicht. Vergleichsweise erhält man z.B. eine Expression von Glucose-Dehydrogenase I in E. coli ohne λ-P_L-Promotor von durchschnittlich 0,1-0,2 Units/ml Kultur, mit dem λ-P_L-Promotor von durchschnittlich 40-50 Units/ml Kultur. Die Effektivität des erfindungsgemäßen Promotors aus Bacillus megaterium im Bacillus-Wirt, vorzugsweise Bacillus megaterium (ca. 35-50 Units/ml Kultur), ist also durchaus vergleichbar mit der des λ-P_L-Promotors in E. coli. Somit kann also auch ein starker Promotor und eine wirksame Regulationseinheit in Bacillus bereitgestellt werden.

Überdies weisen die erfindungsgemäß hergestellten Enzyme eine gute Stabilität auf und lassen sich leicht aus den klaren, farblosen, keine wesentlichen Mengen an Störsubstanzen (z.B. NADH-Oxidasen, Proteasen) enthaltenden Rohextrakten gewinnen.

3

Gegenstand der Erfindung ist somit eine Kontrolleinheit, enthaltend mindestens einen Promotor-Bereich gemäß der DNA-Sequenz I und gegebenenfalls einen Terminator-Bereich gemäß der DNA-Sequenz II.

Die erfindungsgemäße Kontrolleinheit einschließlich des jeweiligen Strukturgen können bekanntermaßen in geeignete Vektoren inseriert und in den Bacillus-Wirt transformiert werden. Als Vektoren sind prinzipiell alle jene geeignet, die durch die Insertion der Fremd-DNA nicht destabilisiert werden, bzw. im Wirtsorganismus selbst nicht Destabilisierungen, beispielsweise durch Deletionen, Rearrangements usw. hervorrufen. Auch sollte die Transformationsrate der Vektor-DNA in dem Bacillus-Wirt möglichst hoch sein, um effizient entsprechend transformierte Klone zu erhalten. Hohe Transformationsraten sind häufig auch ein Zeichen für hohe Kopienzahlen (Amplifikation) des Vektors. Die erfindungsgemäßen Vektoren, vorzugsweise pSA677,- (DSM 4055P) pSAG2 (DSM 4054P) und pSAC4 (DSM 4053P), werden diesen Anforderungen in hervorragender Weise gerecht. So liegt die Transformationsrate beispielsweise für Bacillus megaterium zwischen $10^5$-$10^6$/µg DNA je nach Menge der eingesetzten DNA und dem verwendeten Plasmid. Diese hohen Transformationsraten wurden bislang in Bacillus megaterium nicht erreicht (siehe Brey et al., 1986, J. Bacteriol. 167/2, 623-630) und stellen somit einen erfindungswesentlichen Aspekt dar. Auch bleiben die verwendeten Vektoren im Bacillus-Wirt ohne Selektionsdruck über viele Generationen stabil. Die erfindungsgemäßen Vektoren sind vorzugsweise Schaukelvektoren, d.h. sie enthalten einen Replikationsursprung sowohl für Bacillus als auch für vorzugsweise E. coli. Dabei sind erfindungsgemäß auch solche Schaukelvektoren gemeint, in denen der Bacillus-Replikationsursprung unterschiedlicher Herkunft ist. Dies bietet den Vorteil, daß zu Zwischenklonierungen die bewährte E. coli-Technik eingesetzt werden kann. Solche Schaukelvektoren sind bekannt und in der Literatur beschrieben, z.B. pHV23 und pHV33 (Michel et al., 1980, Gene 12, 147-154) oder pJK302 (Kreft, Hughes, 1982, Curr. Topics Microbiol. Immunol. 96, Springer-Verlag). Erfindungsgemäß sind auch solche Vektoren einsetzbar, die nicht selbst replizierend sind, d.h. die erst nach Integration in das Chromosom oder in andere, einen DNA-Replikationsursprung enthaltende replizierende DNA-Bereiche, wie z.B. kryptische Plasmide, vermehrt werden können.

Gegenstand der Erfindung sind somit auch folgende Plasmide:
- das Plasmid pSA 677 mit der Hinterlegungsnummer DSM 4055P, enthaltend die DNA-Sequenz I und eine DNA-Sequenz, die für das Enzym Glucose-Dehydrogenase-(I) codiert,
- das Plasmid pSAG2 mit der Hinterlegungsnummer DSM 4054P, enthaltend die DNA-Sequenz I und eine DNA-Sequenz, die für das Enzym Glucose-Dehydrogenase-(II) codiert, und
- das Plasmid pSAC4 mit der Hinterlegungsnummer DSM 4053P, enthaltend die DNA-Sequenz I und eine DNA-Sequenz, die für das Enzym Chloramphenicol-Acetyltransferase codiert.

Gegenstand der Erfindung sind ferner folgende Wirtsorganismen:
- der Wirtsorganismus M1296/pSA677 mit der Hinterlegungsnummer DSM 4050, erhalten aus dem Stamm Bacillus megaterium M1296 (DSM 319), transformiert mit dem Plasmid pSA677,
- der Wirtsorganismus M1296/pSAG2 mit der Hinterlegungsnummer DSM 4048, erhalten aus dem Stamm Bacillus megaterium M1296 (DSM 319), transformiert mit dem Plasmid pSAG2 und
- der Wirtsorganismus M1296/pSAC4 mit der Hinterlegungsnummer DSM 4049, erhalten aus dem Stamm Bacillus megaterium M 1296 (DSM 319), transformiert mit dem Plasmid pSAC4.

Gegenstand der Erfindung ist außerdem ein Bakterien-Klonierungs- und Expressionssystem zur Gewinnung von Proteinen, enthaltend einen transformierbaren, mikrobiellen Wirtsorganismus der Gattung Bacillus, vorzugsweise Bacillus megaterium, und ein selbst replizierendes, in der Wirtszelle stabiles Plasmid, welches einen Promotor und gegebenenfalls einen Terminator aus Bacillus megaterium sowie das zur Replikation bzw. Expression zu bringende Strukturgen, vorzugsweise das Glucose-Dehydrogenase-(I)-Gen, das Glucose-Dehydrogenase-(II)-Gen oder das Chloramphenicol-Acetyltransferase-Gen, enthält.

Gegenstand der Erfindung ist letztlich ein Verfahren zur Herstellung von Proteinen, dadurch gekennzeichnet, daß man das oben genannte Klonierungs- und Expressions-System in einer Nährlösung kultiviert und die zur Expression gebrachten Proteine isoliert.

Die Erfindung wird vorzugsweise wie nachfolgend beschrieben durchgeführt.

Zunächst wird chromosomale DNA aus Bacillus megaterium-Zellen isoliert und aufgereinigt (Beispiel 3). Die Anzucht von Bacillus megaterium-Zellen ist in Beispiel 2 näher erläutert. Die so isolierte chromosomale DNA wird mit Hilfe von hierfür geeigneten, üblichen, bekannten Restriktionsendonucleasen in an sich bekannter Weise partiell hydrolysiert. Vorzugsweise wird hierfür das Restriktionsenzym Sau3A eingesetzt. Die Hydrolyse wird bevorzugt so gesteuert, daß hauptsächlich Fragmente mit einer Länge zwischen 9 und 22 Kilobasen (kb), vorzugsweise mehr als 14 kb erhalten werden. Zum Anlegen einer Genbank dient vorzugsweise der bekannte und für diese Zwecke häufig verwendete Phagenvektor λ-EMBL3 (Frischauff et al., 1983, J. Mol. Biol. 170, 827-842). Die Phagen werden vorzugsweise nach der Methode von Arber et al., (1983, in: Lambda II, 433-466, Cold Spring Harbor, New York) präpariert und die Phagen-DNA wird, wie in Beispiel 4 näher beschrieben, präpariert und nach bekannter Weise mittels üblicher Restriktionsendonucleasen, vorzugsweise BamHI, aufgeschnitten. Die Größe der jeweilig enthaltenen DNA-Fragmente wird mittels Gelelektrophorese bestimmt.

In die Schnittstelle der Phagen-DNA werden nun die passenden DNA-Fragmente aus dem Bacillus-Genom mittels käuflicher Ligierungsenzyme, vorzugsweise T4-DNA-Ligase, in an sich bekannter Weise eingebaut (Weiss et al., 1968, J. Biol. Chem. 243, 4543-4555). Die so erhaltenen verschiedenen neukombinierten Phagen-Moleküle werden in einem zu ihrer Vermehrung befähigten Wirtsorganismus, vorzugsweise E. coli, insbesondere E. coli NM539 (ATCC 35639) transduziert (Anzucht E. coli: Beispiel 1) und in an sich bekannter Weise kloniert (Arber et al., 1983, l.c.). Die Transformation von Bakterienzellen mit Plasmiden wird bevorzugt nach der bekannten Calciumchlorid-Methode (Cohen et al., 1972, Proc. Natl. Acad. Sci. USA, 69, 2110-2114) vorgenommen.

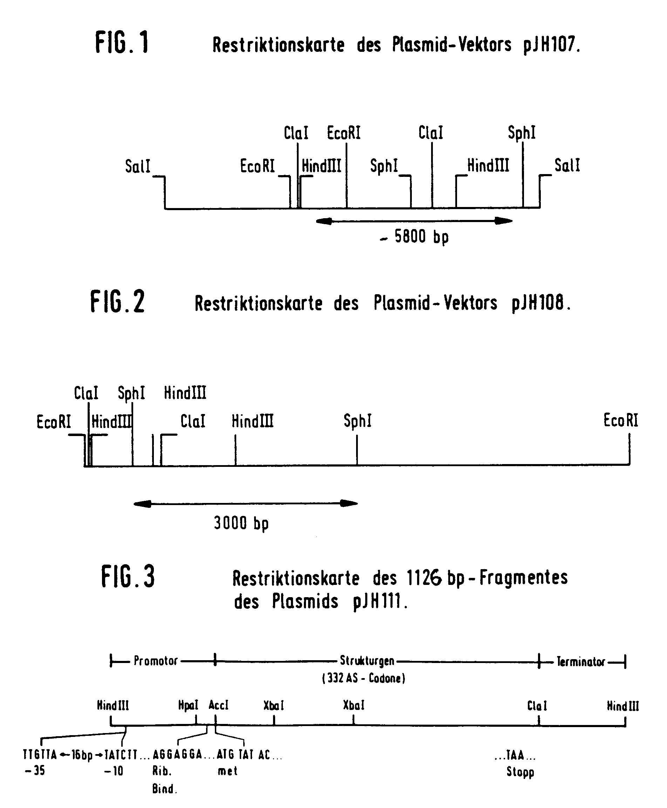

Aus der so angelegten Bacillus megaterium-Genbank werden jene Phagenklone identifiziert, die die genetische Information des in Bacillus-Arten auftretenden NAD/NADP-anhängigen Enzyms Glucose-Dehydrogenase (E.C. 1.1.1.47), im folgenden Glucose-DH genannt, enthalten. Dies geschieht durch Nachweis der Enzymaktivität im Lysat der neuentstandenen Phagenklone mit Hilfe eines spezifischen Filtertests, wobei die Umsetzung von ß-Glucose zu Gluconolacton unter katalytischer Wirkung der Glucose-DH und unter Bildung von $NADH_2$ ausgenutzt wird. Die Bildung von $NADH_2$, die von der Glucose-DH-Bildung abhängig ist (positive Reaktion), wird durch das Auftreten von Fluoreszenzsignalen der auf Filterpapier aufgebrachten Phagenproben (Plaques) nachgewiesen (Beispiel 5). Aufgrund dieser Tests, die auch in den folgenden Arbeitsschritten zur Anwendung kommen, können schließlich Phagenklone mit reproduzierbarer positiver Reaktion identifiziert werden. Um eine Konzentrierung der Glucose-DH-DNA innerhalb der rekombinanten Phagen-DNA zu erreichen, erfolgt in einem weiteren Arbeitsgang die Subklonierung vorzugsweise in Plasmiden. Hierzu wird wiederum die rekombinante Phagen-DNA partiell mit Restriktionsendonucleasen, vorzugsweise Sau3A, wie üblich hydrolysiert, so daß Fragmente von etwa 4-9 kb Größe entstehen. Als Ausgangsplasmid kann im Prinzip jedes Plasmid verwendet werden, welches bei einem Einbau von DNA-Fragmenten der angegebenen Größe nicht destabilisiert wird. Besonders hierfür geeignet ist pBR322 (Bolivar et al., 1977, Gene 2, 95-113), welches vorzugsweise mit der Restriktionsendonuclease BamHI verdaut wird. Die Phagen-DNA-Fragmente werden nun in an sich bekannter Weise mit dem aufgeschnittenen Plasmid-Vektor ligiert (Weiss et al., l.c.). Mit den Ligierungsprodukten wird ein beliebiger, zur Transformation geeigneter, in einer anerkannten Hinterlegungsstelle erhältlicher Wirtsorganismus, vorzugsweise E. coli, in üblicher Weise transformiert. Die so erhaltenen Kolonien werden dem Glucose-DH-Enzymtest (Beispiel 5) unterworfen. Eines der zugrundeliegenden Plasmide besitzt eine Größe von 10.2 kb und wird mit pJH 107 bezeichnet. Zum Zwecke der Restriktionsanalyse wird das Plasmid in an sich bekannter Weise durch die Restriktionsendonucleasen EcoRI, SalI, SphI, HindIII und ClaI vollständig hydrolysiert und die DNA-Fragmente mittels einer Agarosegelelektrophorese in an sich bekannter Weise aufgetrennt. Die Größe des Fragmentes, welches die Glucose-DH-Sequenz enthält, wird mit 5800 bp ermittelt. Figur 1 gibt die Restriktionskarte des erfindungsgemäßen Plasmids pJH107 wieder.

Zur weiteren Eingrenzung des Gens auf dem rekombinanten Plasmid wird vorzugsweise, wie im folgenden skizziert, mit bekannter Methodik vorgegangen:

- Verdauung von pJH107 mit vorzugsweise SphI.
- Einbau von pJH 107 in pBR322 und Transformation eines beliebigen vorzugsweise E. coli-Wirtes. Daraus resultiert der Plasmid-Vektor pJH 108. Dieser enthält ein ca. 3000 bp großes Passagier-DNA-Fragment (Figur 2). Bei der Restriktionsanalyse werden vorzugsweise die Endonucleasen SphI, ClaI, EcoRI und HindIII eingesetzt.
- Verdauung von pJH108, vorzugsweise mit HindIII.
- Einbau in den Plasmid-Vektor pBR322 und Transformation und Klonierung eines Wirtes, vorzugsweise E. coli RR1. Neukombinierter erfindungsgemäßer Plasmid-Vektor: pJH111 (Figur 3).

Der Plasmid-Vektor pJH111 enthält, wie die Restriktionsanalyse zeigt, nur noch ein 1100 bp großes DNA-Fragment neben dem 5500 bp großen Rest-Plasmid. Die entsprechenden E. coli-Klone zeigen Glucose-DH-Enzymaktivität. Eine weitere Verkleinerung des DNA-Fragmentes führt zu vollständigem Verlust der Enzymaktivität. Das Gen für die Glucose-Dehydrogenase einschließlich seiner Regulationseinheit ist auf dem 1100 bp-Fragment lokalisiert.

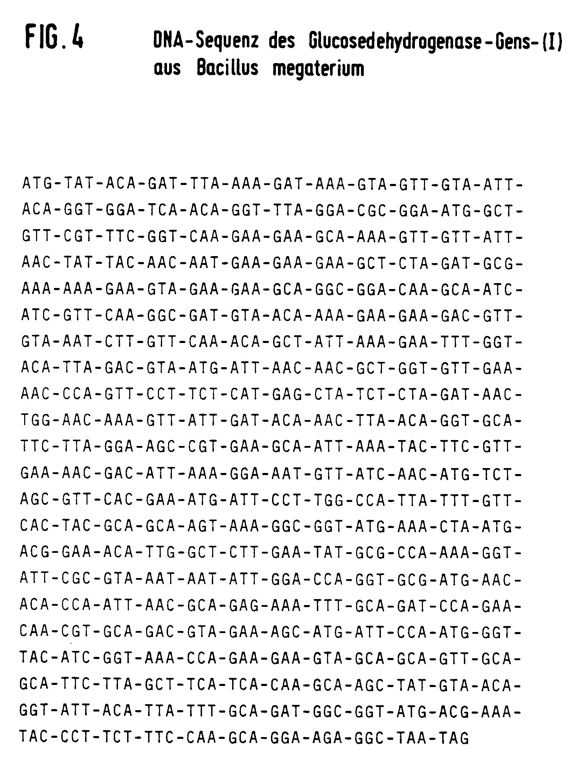

Die Bestimmung der Nukleotid-Sequenz dieses 1100 bp-Fragmentes (z.B. nach Maxam und Gilbert, Methods Enzymol. (1980) 65, 499-580) ergibt die erfindungsgemäße DNA-Sequenz I für den Promotor-Bereich, die erfindungsgemäße DNA-Sequenz II für den Terminator-Bereich und die DNA-Sequenz für das Strukturgen, welches für das Enzym Glucose-DH codiert (Figur 4).

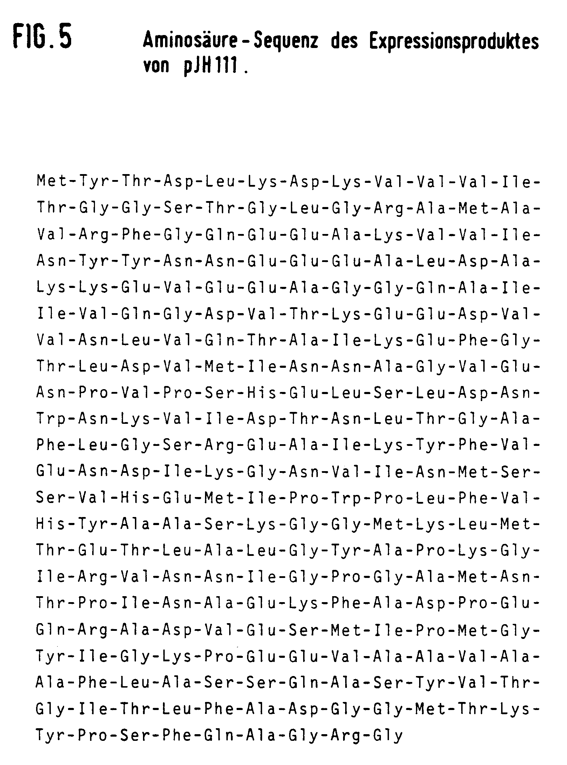

Überraschenderweise weicht die DNA-Sequenz aus Figur 4 und die daraus abgeleitete und durch Proteinsequenzierung des aufgereinigten Enzyms bestätigte Aminosäure-Sequenz (Figur 5) zu fast 20 % von der bekannten Sequenz käuflicher Glucose-DH (Jany et al., 1984, FEBS Lett. 165, 6-19) ab. Sie wird

EP 0 285 949 B1

vor- und nachstehend als Glucose-DH-(I) bezeichnet.

Die Identifizierung der ebenfalls in Bacillus aufgetretenen bekannten Glucose-DH (hier mit Glucose-DH-(II) bezeichnet) erfolgt durch Hybridisierung filtergebundener DNA aus Bacillus megaterium mit dem radioaktiv markierten 1126 bp HindIII-Fragment des Gens. Zu diesem Zweck wird in an sich bekannter Weise chromosomale DNA vorzugsweise mit der Restriktionsendonuklease HindIII hydrolysiert, gemeinsam mit DNA-Längenstandards auf ein 1 % Agarosegel aufgebracht und auf Nitrocellulose übertragen. Als Längenstandard dient neben der durch EcoRI hydrolysierten SPP1 DNA ein Gemisch aus pJH108 (SalI-SphI hydrolysiert) und pJH111 (HindIII hydrolysiert), wodurch eine Größenbestimmung hybridisierender Fragmente nach der Autoradiographie ermöglicht wird. Hybridierungstechnik, radioaktive Markierung und Autoradiographie sind Stand der Technik (z.B. nach Maniatis et al., 1975, Proc. Natl. Acad. Sci. USA 72, 1184-1188; Casey und Davidson, 1977, Nucl. Acids Res. 4, 1539-1532 ; Hanahan und Meselson, 1980, Gene 10, 63-67; Rigby et al., 1977, J. Mol. Biol. 113, 237-251) bzw. für den Fachmann in einfacher Weise daraus ableitbar. Aus dem Autoradiogramm können neben der der eingesetzten Gensonde entsprechenden 1100 bp intensiven Bande schwächere Banden bei 2100 und 5000 bp identifiziert werden. Damit ist gezeigt, daß Bacillus megaterium mehr als eine Glucose-Dehydrogenase enthält.

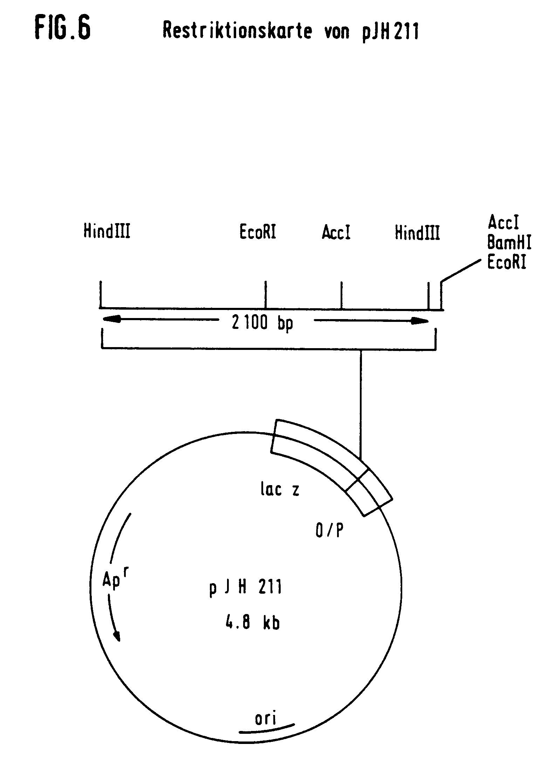

Die Klonierung einer weiteren Glucose-DH entsprechend der Fragmente zwischen 1300 und 5000 bp wird prinzipiell wie oben für das 1100 bp-Fragment ausführlich beschrieben durchgeführt. Als Vektor dient vorzugsweise das E. coli-Plasmid pUC18 (Herkunft: Viera et al., 1982, Gene 19, 259). Man erhält schließlich das neue Plasmid pJH211 (Figur 6) welches ein 2100 bp DNA-Fragment enthält und das in E. coli zur Expression von Glucose-DH führt (das gleichfalls mit der Gensonde hybridisierende 5000 bp-Fragment kann hierbei nicht erhalten werden). In an sich bekannter Weise, bzw. analog zum oben beschriebenen 1100 bp-Fragment, wird die DNA- bzw. Proteinsequenz des zweiten Glucose-DH-Gens ermittelt. Sie entspricht dabei vollkommen der bekannten Sequenz der Glucose-DH.

Die beiden erfindungsgemäßen DNA-Sequenzen I und II sind, wie die Ergebnisse zeigen, nur im 1100 bp-Fragment des Plasmids pJH111 enthalten. Beide Glucose-DH-Gene führen unter Kontrolle ihrer Regulationseinheiten in E. coli zu einer vergleichsweise niedrigen Expression von Glucose-DH (ca. 0,1 Units/ml Kultur). Um eine Expression von Glucose-DH oder auch anderen Strukturgenen in Bacillus, vorzugsweise Bacillus megaterium, zu erhalten, müssen die Plasmide pJH111 und pJH211, insbesondere das die erfindungsgemäße Regulationseinheit enthaltende Plasmid pJH111, umkonstruiert werden.

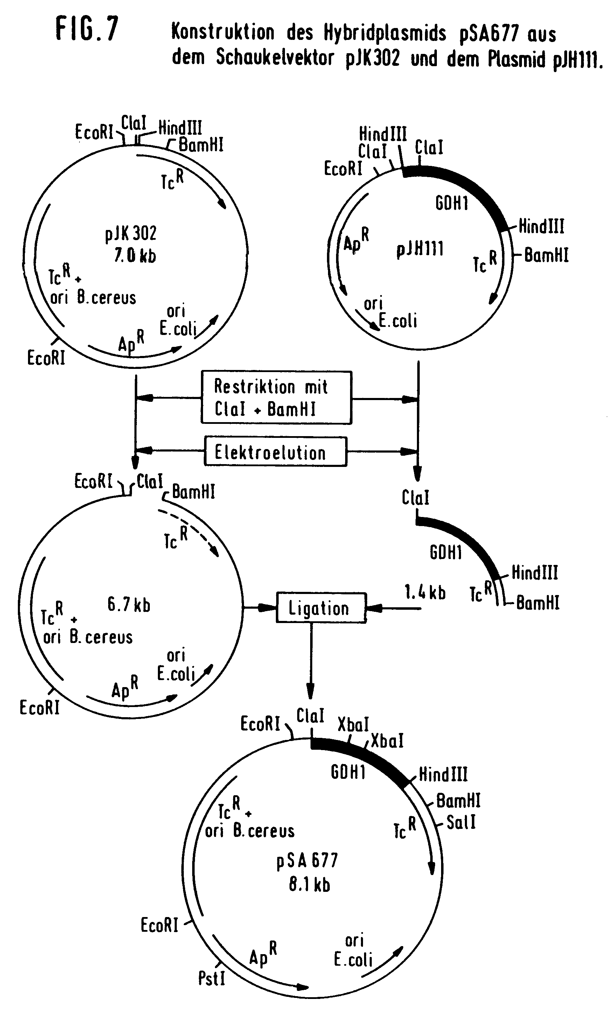

Beide Plasmide enthalten nämlich nur einen Replikationsursprung für E. coli, sind also in Bacillus nicht vermehrbar. Um dies zu ermöglichen, wird auf einen Vektor zurückgegriffen, der sowohl für Bacillus als auch für einen anderen Wirt, vorzugsweise E. coli, einen Replikationsursprung enthält. Dabei ist es erfindungsgemäß gleichgültig, ob der Bacillus-Ori aus Bacillus subtilis, Bacillus cereus, Bacillus megaterium oder anderen Bacillus-Arten oder anderen Gram-positiven Organismen stammt. Zur Herstellung des erfindungsgemäßen Plasmids pSA677 (DSM 4055P) wird vorzugsweise der Schaukelvektor pJK302 (Kreft, Hughes, l.c.) verwendet. pSA677 enthält neben den Gen-Abschnitten, die für die Ampicillin- und Tetracyclin-Resistenz codieren, und den Oris für E. coli und Bacillus cereus, die Promotor-Region gemäß der DNA-Sequenz I sowie die DNA-Sequenz des Glucose-DH-Gens-(I) gemäß Figur 4. Die Konstruktion des Plasmids ist in Figur 7 und Beispiel 6 näher erläutert.

Nach Transformation des pSA677 in Bacillus megaterium werden tetracyclinresistente und Glucose-DH-positive Klone in großer Zahl erhalten. Der Nachweis der Glucose-DH-(I) und Berechnung der Enzymaktivität erfolgt vorzugsweise nach der in Beispiel 5 erläuterten Methode. Dabei werden die oben angegebenen hohen Expressionsraten erzielt.

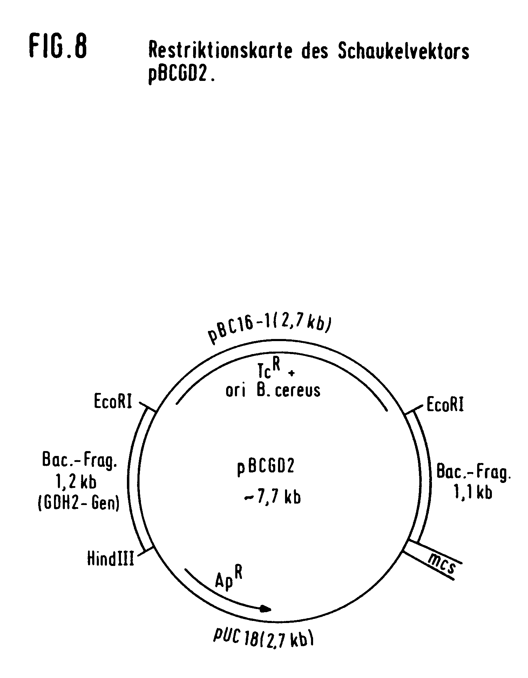

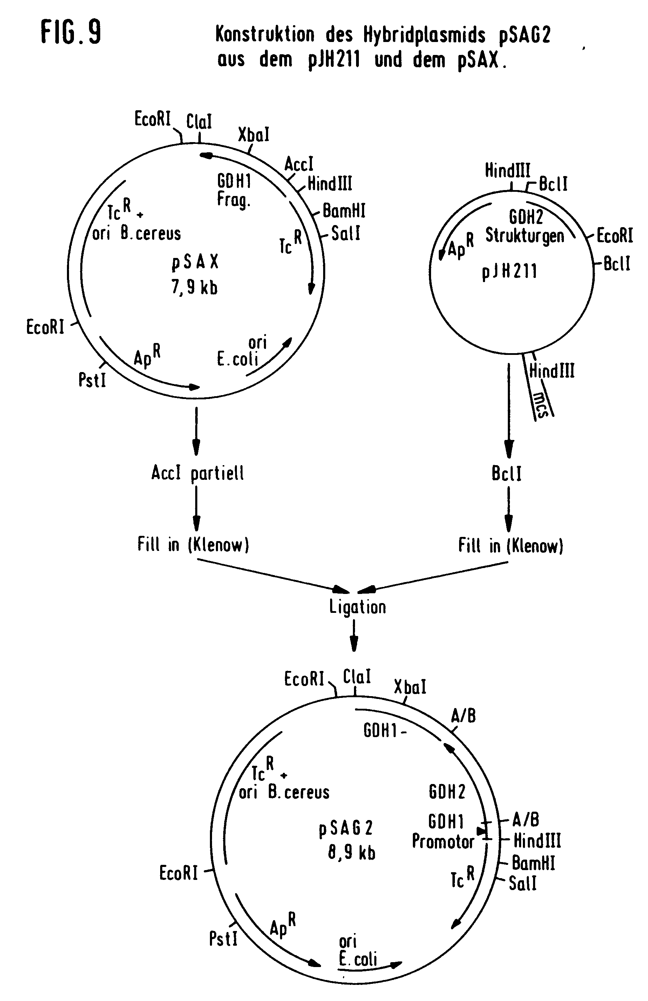

Um auch das Glucose-DH-(II)-Gen in Bacillus, vorzugsweise Bacillus megaterium, zur Expression bringen zu können, wird in das Plasmid pJH211 der bekannte Plasmidvektor pBC16-1 (Bernhard et al., 1978, J. Bacteriol. 133, 897-903), welcher einen Bacillus cereus-Ori und Tetracyclinresistenz besitzt, einkloniert, und der daraus resultierende neue Schaukelvektor pBCGD2 in Bacillus, vorzugsweise Bacillus megaterium, transformiert. Anstelle des pBC16-1 kann auch beispielsweise ein entsprechendes DNA-Fragment mit einem Bacillus megaterium-Ori eingesetzt werden. Die Restriktionskarte des pBCGD2 ist in Figur 8 wiedergegeben. Im Gegensatz zum Glucose-DH-(1)-Gen zeigt das Glucose-DH-(II)-Gen eine deutlich geringere Expression; gegenüber Wildstämmen ist diese nur unwesentlich erhöht (siehe oben). Der Grund hierfür ist darin zu sehen, daß das Plasmid pJH211 nicht die erfindungsgemäße Regulationseinheit enthält, insbesondere den starken Promotor gemäß der DNA-Sequenz I des 1126 bp-Fragmentes im Plasmid pJH111. Bringt man nämlich das Glucose-DH-(II)-Gen unter die Kontrolle des erfindungsgemäßen Promotors, so erhält man wieder sehr hohe Enzymaktivitäten. Dies kann beispielsweise dadurch erreicht werden, daß die erfindungsgemäße DNA-Sequenz I aus dem 1126 bp-Fragment des pJH111 oder des pSA677 mittels geeigneter Restriktionsendonukleasen herausgeschnitten wird und unmittelbar mit dem Strukturgen (hier Glucose-DH-(II)) ligiert wird. Eine andere hier bevorzugte Verfahrensweise besteht darin,

6

daß aus dem erfindungsgemäßen Plasmid pSA677 ein entsprechend der verfügbaren Restriktionsendonucleasen großes Fragment, vorzugsweise das 180 bp große XbaI-Fragment (siehe Figur 3) in an sich bekannter Weise eliminiert wird. Von dieser Deletion sind so viele Aminosäuren betroffen, daß kein funktionsfähiges Glucose-DH-(I)-Protein gebildet werden kann. Transformanten, die das daraus resultierende neue Plasmid pSAX (siehe Figur 9) enthalten, zeigen eine Glucose-DH-(I) negative Reaktion. pSAX wird nun nach bekannten Methoden mit AccI partiell aufgeschnitten und in die Schnittstelle ein entsprechendes, das Glucose-DH-(II)-Gen enthaltende Fragment in üblicher Weise einkloniert. Die Konstruktion des daraus resultierenden neuen Plasmids pSAG2 ist in Figur 9 und Beispiel 7 näher erläutert.

Der erfindungsgemäße Promotor des Glucose-DH-(I)-Gens bewirkt also auch eine starke Expression des homologen Glucose-DH-(II)-Gens (Aktivitäten siehe oben) in einem Bacillus megaterium-Wirt.

Als Beispiel für ein heterologes Gen kann das Chloramphenicol-Acetyltransferase-Gen (CAT) dienen. Dieses Enzym katalysiert die Acetylierung und damit die Inaktivierung des Antibiotikums Chloramphenicol. Plaziert man hinter einen starken Promotor eine solche Resistenzmarke, so sollten die Transformanten leicht zu selektieren sein, da sie gegen hohe Konzentrationen des Antibiotikums resistent sind. Erfindungsgemäß wird das CAT-Gen unter die Kontrolle des starken Promotors gemäß der DNA-Sequenz I gestellt. Dies geschieht vorzugsweise dadurch, daß man das CAT-Gen unmittelbar am Beginn des Glucose-DH-I-Strukturgens inseriert. Letzteres kann somit nicht mehr exprimiert werden. Es ist aber auch bei Verfügung entsprechender Restriktionsendonucleasen denkbar, das CAT- oder allgemein das Fremdgen unmittelbar im Anschluß an den erfindungsgemäßen Promotor zu plazieren und die weitere Expression durch entsprechende Maßnahmen, z.B. durch Einbau eines Stopkodons, zu unterbinden, oder das Glucose-DH-(I)-Strukturgen ganz zu entfernen und das resultierende Plasmid entsprechend aufzufüllen, oder aber das Glucose-DH-(I)-Gen anderweitig zu desaktivieren.

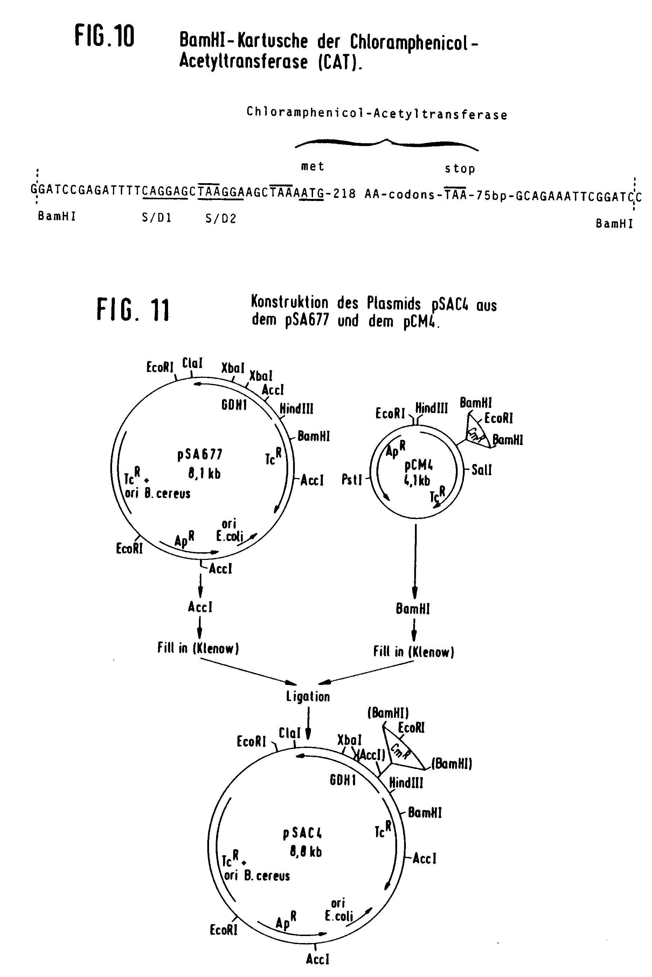

Zur Erstellung der neuen Konstruktion wird vorzugsweise das bekannte Plasmid pCM4 (Close und Rodriguez, 1982, Gene 20, 305-316) benutzt, welches eine sogenannte BamHI-Kartusche des CAT-Gens enthält (Figur 10). Die Konstruktion des neuen erfindungsgemäßen Plasmids pSAC4, welches das CAT-Gen und den erfindungsgemäßen Promotor-Bereich der DNA-Sequenz I enthält, aus pCM4 und pSA677 ist in Figur 11 und Beispiel 8 näher erläutert. Transformiert man pSAC4 beispielsweise in Bacillus megaterium, so verleiht es dem Bacillus megaterium-Rezipienten eine Resistenz gegen 50-100 $\mu$g Chloramphenicol/ml. Nichttransformierte Stämme wachsen dagegen bereits in ca. 2 $\mu$g/ml nicht mehr an. Bacillus-Stämme, die mit dem heterologen Chloramphenicol-Acetyltransferase-Gen transformiert werden, erlangen so eine Resistenz gegenüber Chloramphenicol, die 30-50fach über der ursprünglichen Letaldosis liegt. Die in Bacillus megaterium nach der bevorzugten Methode gebildete Chloramphenicol-Acetyltransferase ist um 13 Aminosäuren größer als die in E. coli (Bildung eines Fusionsproteins, ausgehend von der Ribosomenbindungsstelle des Glucose-DH-(I)-Gens). Die Enzymaktivität der so gebildeten Chloramphenicol-Acetyltransferase in Bacillus megaterium liegt zwischen 500 und 1500, vorzugsweise zwischen 600 und 1300 mUnits/ml Kultur. Die Messung der Enzymaktivität erfolgt vorzugsweise spektralphotometrisch nach bekannter Methodik (Methods of Enzymology, (1980) Vol. 18, S. 742-743). Die nach der bevorzugten Methode transformierten Bacillus-Wirtsorganismen zeigen keine Glucose-DH-Aktivität mehr.

Die Promotor-Region gemäß der DNA-Sequenz I und gegebenenfalls des für die Gesamtregulation wichtigen Terminator-Bereichs gemäß der DNA-Sequenz II sind erfindungsgemäß in der Lage, neben den Glucose-DH-(I/II)-Genen und dem CAT-Gen auch andere homologe oder heterologe Gene, wie die oben beispielhaft genannten Gene, vorzugsweise im Bacillus-Wirt, insbesondere Bacillus megaterium, in guten Ausbeuten zu exprimieren.

In der Beschreibung, in den Beispielen und Figuren werden, sofern dort nicht näher erläutert, folgende Abkürzungen verwendet:

| | |
|---|---|
| A | Adenin |
| $A_{578}$ | Absorption bei 578 nm |
| $A_{578}$-E | Absorptionseinheit bei 578 nm |
| $Ap^R$ | Ampicillinresistenz |
| APS | Ammoniumperoxodisulfat |
| b | Base |
| Bis | N,N,N′,N′-Methylenbisacrylamid |
| bp | Basenpaar |
| BSA | Rinderserumalbumin |
| C | Cytosin |
| CAT | Chloramphenicol-Acetyltransferase-Gen |
| Da | Dalton (g/mol) |
| DNA | Desoxyribonukleinsäure |

| DTT | Dithiothreitol |
|---|---|
| EDTA | Ethylendiamintetraessigsäure |
| E. coli | Escherichia coli |
| G | Guanin |
| galK | Galaktokinase |
| k | x 10$^3$ |
| l | Liter |
| NAD | Nicotinamid-adenin-dinukleotid |
| NADP | Nicotinamid-adenin-dinukleotid-phosphat |
| OD | optische Dichte |
| PEG | Polyethylenglykol |
| rpm | Umdrehungen pro Minute |
| s | Sekunde |
| SDS | Natriumdodecylsulfat |
| T | Thymin |
| T4 | Bakteriophage T4 |
| Tc$^R$ | Tetracyclinresistenz |
| TEMED | N,N,N′,N′-Tetramethylethylendiamin |
| Tris | Tris-(hydroxymethyl)-aminomethan |
| tRNA | Transfer-Ribonukleinsäure |
| U | Einheit der Enzymaktivität (Unit) |

Im folgenden sind die Abbildungen erläutert, in denen einzelne Teilbereiche der Erfindung dargestellt sind:

Figur 1: Restriktionskarte des Plasmid-Vektors pJH107. Eingezeichnet sind die eindeutig zugeordneten Restriktionsschnittstellen. Die Größe der Passagier-DNA beträgt ca. 5800 bp. Das Plasmid pJH107 ist konstruiert aus dem BamHI-verdauten Plasmid pBR322 und der Sau3A-hydrolysierten Konstruktion aus chromosomaler DNA und λ-EMBL-3 Phagen-DNA.

Figur 2: Restriktionskarte des Plasmid-Vektors pJH108. Eingezeichnet sind nur die eindeutig zugeordneten Schnittstellen. Die Größe der Passagier-DNA beträgt ca. 3000 bp. Das Plasmid pJH108 ist konstruiert aus einem Fragment von pJH107 (SphI hydrolysiert) und pBR322.

Figur 3: Restriktionskarte des 1126 bp-Fragmentes des Plasmids pJH111. Angegeben sind u.a. die -35 und -10 Regionen des Promotors sowie die Ribosomenbindungsstelle und Start- und Stopkodon. Es sind ferner nur die im Text eine Rolle spielenden Schnittstellen eingezeichnet.

Figur 4: DNA-Sequenz des Glucosedehydrogenase-Gens aus Bacillus megaterium.

Figur 5: Aminosäure-Sequenz des Expressionsproduktes von pJH111.

Figur 6: Restriktionskarte von pJH211. Nur die zur Restriktionsanalyse verwendeten Schnittstellen sind eingezeichnet. Das Plasmid pJH211 ist konstruiert aus pUC18 und HindIII-hydrolysierter chromosomaler DNA.

Figur 7: Konstruktion des Hybridplasmids pSA677 aus dem Schaukelvektor pJK302 und dem Plasmid pJH111 (Text). pSA677 enthält das Glucose-DH-(I)-Gen und die DNA-Sequenz I und ist sowohl in E. coli als auch in Bacillus vermehrbar.

Figur 8: Restriktionskarte des Schaukelvektors pBCGD2. Das Plasmid ist konstruiert aus pJH211 und dem pBC16-1 (Text). pBCGD2 enthält das Glucose-DH-(II)-Gen ohne die Glucose-DH-(I)-Promotor Region.

Figur 9: Konstruktion des Hybridplasmids pSAG2 aus dem pJH211 und dem pSAX. pSAX ist entstanden aus einem verkürzten pSA677 und enthält ein defektes und nicht mehr funktionsfähiges Glucose-DH-(I)-Strukturgen, wohl aber die Promotor-Region gemäß der DNA-Sequenz I. pJH211 enthält das Glucose-DH-(II)-Strukturgen. pSAG2 ist ein Schaukelvektor, in dem das Glucose-DH-(II)-Gen unter der Kontrolle der genannten Promotor-Region steht.

Figur 10: BamHI-Kartusche der Chloramphenicol-Acetyltransferase Gens (CAT). Besonders gekennzeichnet sind die Stopkodone (TAA), das Startkodon des Strukturgens (ATG) sowie die E. coli-Ribosomenbindungsstellen (SD1/SD2).

Figur 11: Konstruktion des Plasmids pSAC4 aus dem pSA677 und dem pCM4. pSA677 enthält das Glucose-DH-(I)-Gen + die Promotor-Region gemäß DNA-Sequenz I, pCM4 enthält innerhalb einer BamHI-Kartusche das CAT-Gen. Im pSAC4 steht das CAT-Gen unter Kontrolle der Promotor-Region gemäß der DNA-Sequenz I. Das Glucose-DH-(I)-Strukturgen wird aufgrund von Insertionsinaktivierung nicht exprimiert.

8

Die folgenden Beispiele sollen die Erfindung näher erläutern. Die Temperaturen sind durchweg in Grad Celsius angegeben.

Beispiel 1 Anzucht von E. coli-Bakterien

a) Zur Präparation von Plasmiden in mg-Mengen erfolgt die Anzucht von E. coli-Bakterien im 1 l Maßstab in Voll-(LB) oder Minimalmedium (M9). Zu diesem Zweck wird 1 l Medium mit 5 ml einer stationären Vorkultur beimpft und unter ständigem Schütteln ca. 18 h bei 37 ° bis zum Erreichen der stationären Wachstumsphase kultiviert.

b) Für die Überexpression der Glucose-Dehydrogenase wird 200 ml LB-Medium mit 2 ml einer bei 28 ° gewachsenen Übernachtkultur beimpft. Die Bakteriensuspension wird daraufhin bei 28 ° und 160 rpm bis zum Erreichen einer optischen Dichte von 0,5 $A_{578}$-E kultiviert. Im Anschluß wird das Kulturgefäß weitere 16 h bei 42 ° inkubiert.

| LB-Medium: | |
|---|---|
| Casein, enzymatisch hydrolysiert | 10 g |
| Hefeextrakt | 5 g |
| NaCl | 5 g |
| Agar (Festagar) | 15 g |
| (Weichagar) | 7,5 g |
| Aqua bidest. | ad 1000 ml |
| pH-Wert mit 1,0 N NaOH auf 7,4 einstellen | |

Antibiotika wurden in folgenden Konzentrationen zugesetzt:

| Ampicillin: | 50 $\mu$g/ml |
|---|---|
| Tetracyclin: | 20 $\mu$g/ml |

| Modifiziertes M9-Medium: | |
|---|---|
| M9-Salze 10-fach konzentriert | 10 ml |
| 20 % Glucoselösung | 5 ml |
| 1 M MgSO$_4$ | 1 ml |
| 10 % Caseinhydrolysat | 5 ml |
| Thiaminlösung (2 mg/ml) | 0,1 ml |
| Aqua bidest. | ad 1000 ml |

| M9-Salze 10-fach konzentriert: | |
|---|---|
| Na$_2$HPO$_4$ x 2 H$_2$O | 75 g/l |
| KH$_2$PO$_4$ | 30 g/l |
| NH$_4$Cl | 10 g/l |
| NaCl | 5 g/l |

Beispiel 2 Anzucht von Bacillus megaterium-Zellen

Zur Präparation von chromosomaler DNA erfolgt die Anzucht von Bacillus megaterium-Bakterien im 10 l Maßstab in Voll-(LB) Medium. Zu diesem Zweck werden 10 l steriles Medium mit 200 ml einer stationären Vorkultur beimpft und unter Rühren (400 rpm) und Belüftung (400 l Luft/h) bei 28° bis zum Erreichen der späten logarithmischen Wachstumsphase kultiviert.

Beispiel 3 Isolierung von Vektor-DNA

a) Isolierung von Phagen-DNA

Die Suspension von durch Ultrazentrifugation gereinigten Phagen wird auf 20 mM EDTA, 0,5 % SDS eingestellt und nach Zugabe von 50 μg/ml Proteinase K 1 h bei 50° inkubiert. Anschließend wird mehrfach mit gleichem Volumen Phenol und mehrfach mit Chloroform/Isoamylalkohol (24:1) extrahiert. Die DNA wird mit Ethanol in bekannter Weise gefällt und durch Zentrifugation 10 min bei 5 000 rpm sedimentiert. Daraufhin wird sie mit 70 % Ethanol gewaschen, getrocknet und in Puffer aufgenommen.

b) Minipräparation von Plasmiden

Um Plasmide in großer Zahl analysieren zu können, wird die Kurzlysatmethode nach Birnboim (Birnboim und Doly, 1979: Nucl. Acid Res. 7, 1513) verwendet.

Je 1,5 ml Zellsuspension aus einer Übernachtkultur wird durch Zentrifugation 1 min bei 15 000 rpm sedimentiert. Nach Zugabe von 100 μl Lösung A (siehe unten) wird 5 min bei 20° inkubiert. Anschließend werden 200 μl Lösung B (siehe unten) zugegeben und die Gefäße werden in Eis gestellt. Nach weiteren 5 min wird Lösung C (siehe unten) zugesetzt und die Proben werden solange in Eis belassen, bis ein unlöslicher Komplex aus chromosomaler DNA, RNA und Protein ausfällt (2-5 min). Nach einer Zentrifugation (15 min) werden die plasmidhaltigen Überstände in neue 1,5 ml-Eppendorf-Kunststoffgefäße übergeführt.

Die DNA wird mit Ethanol 10 min bei minus 20° gefällt und 15 min in der Zentrifuge sedimentiert. Im Anschluß wird sie zweimal mit 70 % Ethanol gespült und (im Exsikkator) getrocknet.

Nach Aufnahme in 40 μl TE-Puffer (siehe unten) kann sie direkt für die Transformation oder für Restriktionsanalysen eingesetzt werden:

| Birnboim-Lösung A: | Birnboim-Lösung B: |
|---|---|
| 25 mM Tris-HCl, pH 8,0 | 0,2 M NaOH |
| 50 mM Glucose | 1 % SDS |
| 10 mM EDTA | |

| Birnboim-Lösung C: | TE-Puffer: |
|---|---|
| 3 M NaOAc/HOAc, pH 4,8 | 10 mM Tris-Base |
| | 1 mM HCl |
| | pH: 8,0 |

c) Präparative Plasmidisolierung

Die Präparation von Plasmiden in mg-Mengen erfolgt mit Hilfe einer limitierten Protoplasten-Lyse durch Detergenzien (Hardies et al., 1979, J. Biol. Chem.254, 5527-5534).

Eine Bakteriensuspension nach Beispiel 1 wird 20 min bei 5 000 rpm und 4° zentrifugiert. Das Sediment wird in 15 ml Saccharoselösung (25 % Saccharose in 50 mM Tris-HCl, pH 8.0) aufgenommen. Nach Zugabe von 3 ml 0,5 M EDTA und 3 ml Lysozymlösung (20 mg/ml Lysozym in 50 mM Tris-HCl, pH 8,0) wird 30-40 min in Eis inkubiert. Danach werden 2 ml einer 2:1 (v/v) Mischung aus 10 % Polyethylenglykolmonolaurylether/10 % Desoxycholat zugegeben. Die Klärung des Zellaufschlusses erfolgt durch Zentrifugation (30 min) bei 40 000 rpm und 4°. Der klare Überstand wird dekantiert, 100 μg/ml RNase A zugegeben und 45 min in Eis inkubiert. Zur DNA-Fällung wird der RNase-behandelte Überstand mit einem halben Volumen 30 % PEG 6 000 in 1,5 M NaCl versetzt und weitere 30 min in Eis belassen. Man zentrifugiert 20 min bei 8 000 rpm und 4°. Der Niederschlag wird in 5 ml TE-Puffer aufgenommen, 50

μg/ml Proteinase K zugegeben und für 60 min bei 37° inkubiert. Danach wird mehrfach mit TE-gesättigtem Phenol und mehrfach mit Chloroform/Isoamylalkohol (24:1) extrahiert. Die DNA wird mit Ethanol gefällt, gewaschen und getrocknet. Das Sediment wird in 1-2 ml TE-Puffer gelöst und die Plasmidausbeute spektralphotometrisch bei 260 nm bestimmt.

Beispiel 4: Präparation chromosomaler DNA aus Glucose-DH-bildenden Bacillus megaterium-Zellen

Bakterienzellen aus 100 ml einer in Vollmedium gewachsenen stationären Kultur werden 10 min bei 5 000 rpm und 4° sedimentiert. Anschließend wird das Sediment in 10 ml Lysepuffer (50 mM NaCl, 50 mM EDTA, 30 mM Tris-HCl, pH 7.9) aufgenommen; 40 mg Lysozym werden zugegeben und 45 min bei 37° inkubiert. Die Suspension wird auf 1 % SDS eingestellt, mit 5 mg Proteinase K (Hersteller: E. Merck, Darmstadt) versetzt und weitere 30 min bei 37° belassen. Danach wird mehrfach mit TE-gesättigtem Phenol und mehrfach mit Chloroform/Isoamylalkohol (24:1) extrahiert. Die Nukleinsäuren werden in üblicher Weise gefällt, gewaschen und getrocknet.

Zur Hydrolyse noch vorhandener RNA wird das Sediment vorsichtig in 5 ml TE-Puffer resuspendiert, 10 μg/ml RNase zugefügt und 30 min bei 37° inkubiert.

Die Lösung wird daraufhin auf 1 % SDS und 50 μg/ml Proteinase K eingestellt und weitere 30 min bei 37° behandelt. Anschließend wird wie oben beschrieben mit Phenol und Chloroform/Isoamylalkohol extrahiert und gegen TE-Puffer dialysiert. Die Ausbeute an chromosomaler DNA beträgt bei dieser Methode zwischen 2 und 4 mg.

Beispiel 5: Enzymtest für die Glucose-DH

Für diesen Test wird nicht-fluoreszierendes Chromatographiepapier mit 92 ml Indikatorlösung gleichmäßig besprüht und im Anschluß bei 20° getrocknet.

| Indikatorlösung: | |
|---|---|
| 60,5 ml | Natriumphosphat-Puffer (0,12 M; pH 7,6) |
| 30 ml | D-Glucose-Lösung (10 % w/v) |
| 1,5 ml | $NAD^+$-Lösung (70 ng/ml) |

Das präparierte Chromatographiepapier wird vor der Durchführung des Enzymtests auf die Größe der Nährbodenplatten zurechtgeschnitten (Durchmesser ca. 8 cm).

Zur Durchführung des Tests bei Phagenlysaten werden die Platten sofort nach Bildung gut sichtbarer Plaques (600-800 pro Platte) dem Brutschrank entnommen. Zunächst werden die Petrischalen markiert und identisch gekennzeichnete Testfilter deckungsgleich aufgelegt. Nach 2 min werden sie abgenommen, im Heißluftstrom getrocknet und im Anschluß unter Licht der Wellenlänge 366 nm betrachtet. Wurde aktive Glucose-DH auf die Test-Filter übertragen, so läßt sich an diesen Stellen $NAD^+$ zu NADH reduzieren, welches durch Fluoreszenz deutlich wahrgenommen werden kann. Mit Hilfe der Filtermarkierungen werden daraufhin positive Phagenklone auf der Nährbodenplatte identifiziert und isoliert.

Zur Durchführung des Tests bei Bakterienklonen werden Zellen in identischer Weise auf zwei Nährbodenplatten übertragen (100-200 Kolonien pro Platte). Während eine der Platten als Referenzplatte dient ("master plate"), wird die andere für den Enzymtest verwendet. Dabei werden die Bakterien auf Aluminiumfolie transferiert und ca. 5 μl der Aufschlußlösung auf jede Kolonie pipettiert. Zur Lyse werden die Zellen 30 min bei 20° in einer feuchten Kammer inkubiert. Anschließend wird, wie oben beschrieben, ein Testfilter aufgelegt, getrocknet und die Stelle der enzymatischen Reaktion identifiziert. Die entsprechenden positiven Bakterien der Referenzplatte können im Anschluß zur Plasmidisolierung (Beispiel 3) verwendet werden.

| Aufschlußlösung: | |
|---|---|
| 100 mM | Natriumphosphat-Puffer, pH 6.5 |
| 20 mM | EDTA |
| 5 mg/ml | Lysozym |

Die Aktivitätsbestimmung erfolgt am Spektralphotometer bei 25°.

11

| Testlösung: | |
|---|---|
| 120 mM | Natriumphosphat-Puffer, pH 7.6 |
| 100 mM | Glucose |
| 2 mM | NAD$^+$ |

Die Angabe der Enzymaktivität (U/ml) erfolgt in $\mu$Mol Substratumsatz pro min.

Beispiel 6: Konstruktion des Plasmids pSA677, enthaltend das Glucose-DH-(I)-Gen und die Promotor-DNA-Sequenz I.

Ausgangsplasmide sind der Schaukelvektor pJK302 und der erfindungsgemäße Plasmidvektor pJH111. pJK302 ist konstruierbar aus pBR322 (Bolivar et al., 1977, Gene 2, 95-113) und pBC16-1 (Bernhard et al., 1978, l.c.). Die Konstruktion wird im Detail bei Kreft und Hughes (1982), l.c., beschrieben. Die Konstruktion des pJH111 ist dem Text zu entnehmen. Aus dem Plasmid pJK302 wird mit den entsprechenden Restriktionsendonucleasen ein ca. 0,3 kb großes DNA-Stück zwischen der ClaI- und der BamHI-Schnittstelle in bekannter Weise herausgeschnitten. Mit vorzugsweise denselben Restriktionsendonucleasen wird aus dem erfindungsgemäßen Plasmid pJH111 ein etwa 1,4 kb-Fragment herausgeschnitten. Dieses enthält u.a. das Glucose-DH-(I)-Strukturgen und den erfindungsgemäßen starken Promotor gemäß der DNA-Sequenz I. Dieses Fragment wird in an sich bekannter Weise in das pJK302-Restplasmid einkloniert, nachdem die jeweiligen Fragmente zuvor nach Standardverfahren, z.B. durch Elektroelution aus den Restriktionsansätzen, isoliert wurden. Die Ligierung erfolgt vorzugsweise mit käuflicher T4-DNA-Ligase (Weiss et al., 1968, J. Biol. Chem. 243, 4543-4555). Das neu entstehende Plasmid pSA677 ist 8,1 kb groß, besitzt einen Replikationsursprung für E. coli und Bacillus, Resistenzmarken für Tetracyclin und Ampicillin sowie die erfindungsgemäße Promotor-Sequenz I und das neue Glucose-DH-(I)-Gen (Figur 4, 5). Zur Zwischenklonierung werden beliebige E. coli-Zellen, beispielsweise E. coli SF8 (Herkunft Struhl et al., 1976, PNAS USA, 73, 1474-1475) mit pSA677 in an sich bekannter Weise z.B. nach der Calciumchlorid-Methode (z.B. Cohen et al., 1972, Proc. Natl. Acad. Sci. USA 69, 2110-2114) transformiert, und die entstandenen Klone auf Glucose-DH-Aktivität mittels des in Beispiel 5 beschriebenen Tests untersucht. Die Transformation im homologen Wirt, z.B. Bacillus megaterium erfolgt nach der Methode von Vorobjeva et al., 1980 (FEMS Microbiol. Lett. 7, 261-263), welche in folgenden Parametern verbessert wurde:

a) Lysozym-Konzentration ca. 0,5 mg/ml
b) Protoplasten-Regeneration in Medium ohne Antibiotikum für 4 bis 5 Stunden
c) Selektion von Transformanten durch Tetracyclin (ca. 12,5 $\mu$g/ml)

Beispiel 7: Konstruktion des Plasmids pSAG2, enthaltend das Glucose-DH-(II)-Gen und die Promotor-DNA-Sequenz I.

Aus dem Plasmid pSA677 wird zunächst in an sich bekannter Weise ein etwa 180 bp großes XbaI-Fragment durch Restriktion und anschließende Ligation eliminiert. Das neu entstehende Plasmid trägt die Bezeichnung pSAX. Entsprechende Transformanten vermögen keine Glucose-DH mehr zu bilden. In das Plasmid pJH211, welches das Glucose-DH-(II)-Strukturgen (ohne den Glucose-DH-(I)-Promotor) enthält, wird nun analog zu Beispiel 6 der Plasmidvektor pBC16-1 in an sich bekannter Weise einkloniert. Daraus resultiert der neue Schaukelvektor pBCGD2 (Figur 8). pBCGD2 wird nun vorzugsweise mit der Restriktionsendonuclease BclI aufgeschnitten und das entstehende Glucose-DH-(II)-Strukturgen und die die Ribosomenbindungsstelle tragenden DNA-Fragmente in die entsprechende AccI-Schnittstelle des pSAX in an sich bekannter Weise einkloniert. Die Restriktion erfolgt dabei vorzugsweise so, daß die Glucose-DH-(II)-Promotor-Sequenz außerhalb des BclI-Fragmentes liegt. Die Restriktionsschnittstellen werden dann bekannterweise mit dem Klenow-Fragment der DNA-Polymerase I und den entprechenden Desoxynukleotiden aufgefüllt. Die Isolierung der entsprechenden Fragmente, ihre Ligierung bzw. Rezirkularisierung erfolgt wiederum nach Standardmethoden. Der neue erfindungsgemäße Vektor pSAG2 enthält das komplette Strukturgen der Glucose-DH-(II), welches unter der Kontrolle der erfindungsgemäßen Promotor-Region gemäß der DNA-Sequenz I steht. Zur Zwischenklonierung dienen wiederum E. coli-Zellen, beispielsweise E. coli SF8 (siehe Beispiel 6), welche mit dem Plasmid pSAG2 in üblicher Weise transformiert werden. Transformation von Bacillus-Zellen siehe Beispiel 6.

Beispiel 8: Konstruktion des Plasmids pSAC4, enthaltend das Chloramphenicol-Acetyltransferase-Gen und die Promotor-DNA-Sequenz I.

Ausgangsplasmide sind das erfindungsgemäße Plasmid pSA677, welches die DNA-Sequenz I aus der Promotor-Region sowie das Glucose-DH-(I)-Strukturgen enthält, sowie das bekannte, käufliche Plasmid pCM4 (Close u. Rodriguez, l.c.). Dieses enthält eine BamHI-Kartusche des CAT-Gens. pSA677 wird in an sich bekannter Weise mit AccI partiell hydrolysiert, pCM4 wird vorzugsweise mit BamHI restringiert. Die Restriktionsschnittstellen werden in üblicher Weise mit dem Klenow-Fragment der DNA-Polymerase I und entsprechenden Desoxynukleotiden aufgefüllt und die Fragmente nach Standardmethoden, z.B. Elektroelution, isoliert. Die Ligierung erfolgt vorzugsweise mit T4-DNA-Ligase (siehe Beispiel 6). Das neue erfindungsgemäße Plasmid pSAC4 enthält somit u.a. die Promotor-Region gemäß der DNA-Sequenz I, das CAT-Gen, sowie ein aufgrund von Insertionsinaktivierung nicht mehr exprimierbares Glucose-DH-(I)-Gen. Außerdem enthält es einen Bacillus-und einen E. coli-Ori sowie Resistenzmarken für Tetracyclin und Ampicillin. Transformation von E. coli- und Bacillus-Zellen mit dem pSAC4 erfolgt vorzugsweise wie in Beispiel 6 angegeben.

Beispiel 9: Expression von Glucose-DH-(I) in Bacillus megaterium M 1296/pSA677 (DSM 4050)

Für die Expression der Glucose-Dehydrogenase werden 10 l Nährlösung (s.u.) mit 200 ml einer bei 28° gewachsenen Übernachtkultur beimpft und unter Rühren (400 rpm) und Belüftung (400 l Luft/h) 24 Stunden bei 28° kultiviert. Nach 18 Std. Wachstum werden in 2stündigem Abstand Proben gezogen:

| Kulturdauer Stunden | pH | OD (λ 578) | Glucose-DH U/ml Kultur | NADH-Oxidase U/ml Kultur | mg Protein/ ml Kultur |
|---|---|---|---|---|---|
| 18 | 6,85 | 2,75 | 31 | 0,002 | ... |
| 20 | 6,6 | 2,83 | 32,75 | 0,003 | ... |
| 22 | 6,6 | 2,81 | 34,5 | 0,004 | ... |
| 24 | 6,4 | 2,80 | 33,2 | 0,004 | 5,9 |

Die Ernte der Zellen erfolgt durch Zentrifugation, die Freisetzung der intrazellulären Enzyme durch Ultraschallaufschluß. Die Aktivitätsbestimmung erfolgt am Spektralphotometer bei 25°.

| Testlösung: | |
|---|---|
| 120 mM | Natriumphosphat-Puffer, pH 7.6 |
| 100 mM | Glucose |
| 2 mM | $NAD^+$ |

Die Angabe der Enzymaktivität (U/ml) erfolgt in $\mu$Mol Substratumsatz pro min.

| Nährlösung: | |
| --- | --- |
| Natriumglutaminat | 5,0 g |
| Cornsteep (Extrakt aus Maisquellwasser) | 5,0 g |
| $(NH_4)2\ HPO_4$ | 2,0 g |
| $MgSO_4$ | 0,1 g |
| $FeSO_4$ | 0,02 g |
| $CaCl_2$ | 0,02 g |
| $NaNO_3$ | 0,1 g |
| Phosphatpuffer 1/30 molar | pH 6,8 |
| Glycerin 87 %ig | 10,0 g |
| Saccharose (getrennt sterilis.) | 10,0 g |
| Leitungswasser | ad 10 l |

**Patentansprüche**

1. Genetische Kontrolleinheit, enthaltend mindestens einen Promotor- und einen Terminator-Bereich, dadurch gekennzeichnet, daß der Bereich, der die Promotor-Region enthält, durch die DNA-Sequenz I:

```
AAG CTT CTC ATT ATC ACA AAA TGG GAA TTG TTA TTT

CAA CGC TTG GCG GTA TCT TCT TAT TAG GAG AAA AGA

AAA CGA AGC GTC AGC TTA TTT CGA TTA CGA TTG GTA

TTG TGC TCA TCA TTT TAG GTG GCG TAT TCC TCG GAA

TGG CTA AAA GTT AAC GTT TAC GTA AAA GTT AAT GAT

AGT ATG TAT GAA TAA ATG AAA ACA CTA GGA GGA TTT

TAT T
```

repräsentiert wird.

2. Genetische Kontrolleinheit nach Anspruch 1, dadurch gekennzeichnet, daß der Bereich, der die Terminator-Region enthält, durch die DNA-Sequenz II:

```
TTT TTA ATC GAT GTA GAA AAA AGA GAC ATG GCA AAA

AGC GCC ATG TCT CTT TTT TCT ATA CCT AAG CGA ATG

ACA CCC CCC TTT CGG GGT TGG TGG CAT TCG CCT AGG

TAA AAG CTT
```

repräsentiert wird.

3. Promotor- und/oder Terminator-Sequenz nach Anspruch 1 und/oder 2, dadurch gekennzeichnet, daß sie aus Bacillus megaterium stammt.

4. Plasmid mit der Bezeichnung pSA677 und der Hinterlegungsnummer DSM4055P, enthaltend die DNA-Sequenz I nach Anspruch 1 und eine DNA-Sequenz, die für das Enzym Glucose-Dehydrogenase (I) codiert.

5. Plasmid mit der Bezeichnung pSAG2 und der Hinterlegungsnummer DSM 4054P, enthaltend die DNA-Sequenz I nach Anspruch 1 und eine DNA-Sequenz, die für das Enzym Glucose-Dehydrogenase (II) codiert.

**6.** Plasmid mit der Bezeichnung pSAC4 und der Hinterlegungsnummer DSM 4053P, enthaltend die DNA-Sequenz I nach Anspruch 1 und die DNA-Sequenz, die für das Enzym Chloramphenicol-Acetyltransferase codiert.

**7.** Wirtsorganismus mit der Bezeichnung M1296/pSA677 und der Hinterlegungsnummer DSM 4050, erhalten aus dem Stamm Bacillus megaterium M1296 (DSM 319), transformiert mit dem Plasmid pSA677.

**8.** Wirtsorganismus mit der Bezeichnung M1296/pSAG2 und der Hinterlegungsnummer DSM 4048, erhalten aus dem Stamm Bacillus megaterium M1296 (DSM 319), transformiert mit dem Plasmid pSAG2.

**9.** Wirtsorganismus mit der Bezeichnung M1296/pSAC4 und der Hinterlegungsnummer DSM 4049, erhalten aus dem Stamm Bacillus megaterium M1296 (DSM 319), transformiert mit dem Plasmid pSAC4.

**10.** Bakterien-Klonierungs- und Expressionssystem zur Gewinnung von Proteinen, enthaltend
a) einen transformierbaren, mikrobiellen Wirtsorganismus der Gattung Bacillus und
b) ein selbst replizierendes, in der Wirtszelle stabiles Plasmid, welches einen Promotor und einen Terminator aus Bacillus megaterium und das zur Replikation bzw. Expression zu bringende Strukturgen enthält.

**11.** System nach Anspruch 10, dadurch gekennzeichnet, daß der Wirtsorganimus Bacillus megaterium ist.

**12.** System nach Anspruch 10 oder 11, dadurch gekennzeichnet, daß die jeweiligen Strukturgene für die Enzyme Glucose-Dehydrogenase I, Glucose-Dehydrogenase II und Chloramphenicol-Acetyltransferase codieren.

**13.** Verfahren zur Herstellung von Proteinen, dadurch gekennzeichnet, daß man ein System nach Anspruch 12 in einer Nährlösung kultiviert und die zur Expression gebrachten Proteine isoliert.

**Claims**

**1.** Genetic control unit containing at least one promoter region and one terminator region, characterized in that the region which contains the promoter region is represented by the DNA sequence I:

```
AAG CTT CTC ATT ATC ACA AAA TGG GAA TTG TTA TTT
CAA CGC TTG GCG GTA TCT TCT TAT TAG GAG AAA AGA
AAA CGA AGC GTC AGC TTA TTT CGA TTA CGA TTG GTA
TTG TGC TCA TCA TTT TAG GTG GCG TAT TCC TCG GAA
TGG CTA AAA GTT AAC GTT TAC GTA AAA GTT AAT GAT
AGT ATG TAT GAA TAA ATG AAA ACA CTA GGA GGA TTT
TAT T
```

**2.** Genetic control unit according to Claim 1, characterized in that the region which contains the terminator region is represented by the DNA sequence II:

```
TTT TTA ATC GAT GTA GAA AAA AGA GAC ATG GCA AAA
AGC GCC ATG TCT CTT TTT TCT ATA CCT AAG CGA ATG
ACA CCC CCC TTT CGG GGT TGG TGG CAT TCG CCT AGG
TAA AAG CTT
```

3. Promoter and/or terminator sequence according to claim 1 and/or 2, characterized in that it originates from Bacillus megaterium.

4. Plasmid, called pSA677 and having the depository number DSM 4055P, containing the DNA sequence I according to claim 1 and a DNA sequence which codes for the enzyme glucose dehydrogenase (I).

5. Plasmid, called pSAG2 and having the depository number DSM 4054P, containing the DNA sequence I according to Claim 1 and a DNA sequence which codes for the enzyme glucose dehydrogenase (II).

6. Plasmid, called pSAC4 and having the depository number DSM 4053P, containing the DNA sequence I according to Claim 1 and the DNA sequence which codes for the enzyme chloramphenicol acetyltransferase.

7. Host organism, called M1296/pSA677 and having the depository number DSM 4050, obtained from the strain Bacillus megaterium M1296 (DSM 319) transformed with the plasmid pSA677.

8. Host organism, called M1296/pSAG2 and having the depository number DSM 4048, obtained from the strain Bacillus megaterium M1296 (DSM 319) transformed with the plasmid pSAG2.

9. Host organism, called M1296/pSAC4 and having the depository number DSM 4049, obtained from the strain Bacillus megaterium M1296 (DSM 319) transformed with the plasmid pSAC4.

10. Bacterial cloning and expression system for obtaining proteins, containing
    a) a transformable microbial host organism of the genus Bacillus and
    b) a plasmid which undergoes autonomous replication and is stable in the host cell and which contains a promoter and a terminator from Bacillus megaterium and the structural gene whose replication and expression is to be brought about.

11. System according to Claim 10, characterized in that the host organism is Bacillus megaterium.

12. System according to Claim 10 or 11, characterized in that the particular structural genes code for the enzymes glucose dehydrogenase I, glucose dehydrogenase II and chloramphenicol acetyltransferase.

13. Process for the preparation of proteins, characterized in that a system according to Claim 12 is cultivated in a nutrient solution, and the proteins whose expression has been brought about are isolated.

**Revendications**

1. Unité de régulation génétique, contenant au moins une région promotrice et une région de terminateur, caractérisée en ce que la zone qui contient la région promotrice est représentée par la séquence d'ADN I:

```
AAG CTT CTC ATT ATC ACA AAA TGG GAA TTG TTA TTT

CAA CGC TTG GCG GTA TCT TCT TAT TAG GAG AAA AGA

AAA CGA AGC GTC AGC TTA TTT CGA TTA CGA TTG GTA

TTG TGC TCA TCA TTT TAG GTG GCG TAT TCC TCG GAA

TGG CTA AAA GTT AAC GTT TAC GTA AAA GTT AAT GAT

AGT ATG TAT GAA TAA ATG AAA ACA CTA GGA GGA TTT

TAT T
```

**2.** Unité de régulation génétique selon la revendication 1, caractérisée en ce que la zone qui contient la région de terminateur est représentée par la séquence d'ADN II:

```
TTT TTA ATC GAT GTA GAA AAA AGA GAC ATG GCA AAA

AGC GCC ATG TCT CTT TTT TCT ATA CCT AAG CGA ATG

ACA CCC CCC TTT CGG GGT TGG TGG CAT TCG CCT AGG

TAA AAG CTT
```

**3.** Séquence promotrice et/ou de terminateur selon la revendication 1 et/ou 2, caractérisée en ce qu'elle provient de *Bacillus megaterium*.

**4.** Plasmide portant la désignation pSA677 et le numéro de dépôt DSM 4055P, contenant la séquence d'ADN I selon la revendication 1 et une séquence d'ADN qui code pour l'enzyme glucose déshydrogénase I.

**5.** Plasmide portant la désignation pSAG2 et le numéro de dépôt DSM 4054P, contenant la séquence d'ADN I selon la revendication 1 et une séquence d'ADN qui code pour l'enzyme glucose déshydrogénase II.

**6.** Plasmide portant la désignation pSAC4 et le numéro de dépôt DSM 4053P, contenant la séquence d'ADN I selon la revendication 1 et une séquence d'ADN qui code pour l'enzyme chloramphénicol acétyltransférase.

**7.** Organisme hôte portant la désignation M1296/pSA677 et le numéro de dépôt DSM 4050, obtenu à partir de la souche *Bacillus megaterium* M 1296 (DSM 319), transformée par le plasmide pSA677.

**8.** Organisme hôte portant la désignation M1296/pSAG2 et le numéro de dépôt DSM 4048, obtenu à partir de la souche *Bacillus megaterium* M 1296 (DSM 319), transformée par le plasmide pSAG2.

**9.** Organisme hôte portant la désignation M1296/pSAC4 et le numéro de dépôt DSM 4049, obtenu à partir de la souche *Bacillus megaterium* M 1296 (DSM 319), transformée par le plasmide pSAC4.

**10.** Système bactérien de clonage et d'expression pour l'obtention de protéines, contenant
a) un organisme hôte microbien transformable appartenant au genre *Bacillus* et
b) un plasmide capable d'autoréplication, stable dans la cellule hôte, qui contient un promoteur et un terminateur provenant de *Bacillus megaterium*, et le gène structural à répliquer ou à exprimer.

**11.** Système selon la revendication 10, caractérisé en ce que l'organisme hôte est *Bacillus megaterium*.

**12.** Système selon la revendication 10 ou 11, caractérisé en ce que les gènes structuraux respectifs codent pour les enzymes glucose déshydrogénase I, glucose déshydrogénase II et chloramphénicol acétyl-

17

transférase.

13. Procédé pour la production de protéines, caractérisé en ce que l'on cultive dans une solution nutritive un système selon la revendication 12, et on isole les protéines exprimées.

## FIG. 1  Restriktionskarte des Plasmid-Vektors pJH107.

~ 5800 bp

## FIG. 2  Restriktionskarte des Plasmid-Vektors pJH108.

3000 bp

## FIG. 3  Restriktionskarte des 1126 bp-Fragmentes des Plasmids pJH111.

# FIG. 4

DNA-Sequenz des Glucosedehydrogenase-Gens-(I) aus Bacillus megaterium

```
ATG-TAT-ACA-GAT-TTA-AAA-GAT-AAA-GTA-GTT-GTA-ATT-
ACA-GGT-GGA-TCA-ACA-GGT-TTA-GGA-CGC-GGA-ATG-GCT-
GTT-CGT-TTC-GGT-CAA-GAA-GAA-GCA-AAA-GTT-GTT-ATT-
AAC-TAT-TAC-AAC-AAT-GAA-GAA-GAA-GCT-CTA-GAT-GCG-
AAA-AAA-GAA-GTA-GAA-GAA-GCA-GGC-GGA-CAA-GCA-ATC-
ATC-GTT-CAA-GGC-GAT-GTA-ACA-AAA-GAA-GAA-GAC-GTT-
GTA-AAT-CTT-GTT-CAA-ACA-GCT-ATT-AAA-GAA-TTT-GGT-
ACA-TTA-GAC-GTA-ATG-ATT-AAC-AAC-GCT-GGT-GTT-GAA-
AAC-CCA-GTT-CCT-TCT-CAT-GAG-CTA-TCT-CTA-GAT-AAC-
TGG-AAC-AAA-GTT-ATT-GAT-ACA-AAC-TTA-ACA-GGT-GCA-
TTC-TTA-GGA-AGC-CGT-GAA-GCA-ATT-AAA-TAC-TTC-GTT-
GAA-AAC-GAC-ATT-AAA-GGA-AAT-GTT-ATC-AAC-ATG-TCT-
AGC-GTT-CAC-GAA-ATG-ATT-CCT-TGG-CCA-TTA-TTT-GTT-
CAC-TAC-GCA-GCA-AGT-AAA-GGC-GGT-ATG-AAA-CTA-ATG-
ACG-GAA-ACA-TTG-GCT-CTT-GAA-TAT-GCG-CCA-AAA-GGT-
ATT-CGC-GTA-AAT-AAT-ATT-GGA-CCA-GGT-GCG-ATG-AAC-
ACA-CCA-ATT-AAC-GCA-GAG-AAA-TTT-GCA-GAT-CCA-GAA-
CAA-CGT-GCA-GAC-GTA-GAA-AGC-ATG-ATT-CCA-ATG-GGT-
TAC-ATC-GGT-AAA-CCA-GAA-GAA-GTA-GCA-GCA-GTT-GCA-
GCA-TTC-TTA-GCT-TCA-TCA-CAA-GCA-AGC-TAT-GTA-ACA-
GGT-ATT-ACA-TTA-TTT-GCA-GAT-GGC-GGT-ATG-ACG-AAA-
TAC-CCT-TCT-TTC-CAA-GCA-GGA-AGA-GGC-TAA-TAG
```

## FIG.5    Aminosäure-Sequenz des Expressionsproduktes von pJH111.

```
Met-Tyr-Thr-Asp-Leu-Lys-Asp-Lys-Val-Val-Val-Ile-
Thr-Gly-Gly-Ser-Thr-Gly-Leu-Gly-Arg-Ala-Met-Ala-
Val-Arg-Phe-Gly-Gln-Glu-Glu-Ala-Lys-Val-Val-Ile-
Asn-Tyr-Tyr-Asn-Asn-Glu-Glu-Glu-Ala-Leu-Asp-Ala-
Lys-Lys-Glu-Val-Glu-Glu-Ala-Gly-Gly-Gln-Ala-Ile-
Ile-Val-Gln-Gly-Asp-Val-Thr-Lys-Glu-Glu-Asp-Val-
Val-Asn-Leu-Val-Gln-Thr-Ala-Ile-Lys-Glu-Phe-Gly-
Thr-Leu-Asp-Val-Met-Ile-Asn-Asn-Ala-Gly-Val-Glu-
Asn-Pro-Val-Pro-Ser-His-Glu-Leu-Ser-Leu-Asp-Asn-
Trp-Asn-Lys-Val-Ile-Asp-Thr-Asn-Leu-Thr-Gly-Ala-
Phe-Leu-Gly-Ser-Arg-Glu-Ala-Ile-Lys-Tyr-Phe-Val-
Glu-Asn-Asp-Ile-Lys-Gly-Asn-Val-Ile-Asn-Met-Ser-
Ser-Val-His-Glu-Met-Ile-Pro-Trp-Pro-Leu-Phe-Val-
His-Tyr-Ala-Ala-Ser-Lys-Gly-Gly-Met-Lys-Leu-Met-
Thr-Glu-Thr-Leu-Ala-Leu-Gly-Tyr-Ala-Pro-Lys-Gly-
Ile-Arg-Val-Asn-Asn-Ile-Gly-Pro-Gly-Ala-Met-Asn-
Thr-Pro-Ile-Asn-Ala-Glu-Lys-Phe-Ala-Asp-Pro-Glu-
Gln-Arg-Ala-Asp-Val-Glu-Ser-Met-Ile-Pro-Met-Gly-
Tyr-Ile-Gly-Lys-Pro-Glu-Glu-Val-Ala-Ala-Val-Ala-
Ala-Phe-Leu-Ala-Ser-Ser-Gln-Ala-Ser-Tyr-Val-Thr-
Gly-Ile-Thr-Leu-Phe-Ala-Asp-Gly-Gly-Met-Thr-Lys-
Tyr-Pro-Ser-Phe-Gln-Ala-Gly-Arg-Gly
```

# FIG.6    Restriktionskarte von pJH 211

**FIG.7**  Konstruktion des Hybridplasmids pSA677 aus dem Schaukelvektor pJK302 und dem Plasmid pJH111.

**FIG.8**  Restriktionskarte des Schaukelvektors pBCGD2.

# FIG.9

Konstruktion des Hybridplasmids pSAG2
aus dem pJH211 und dem pSAX.

## FIG.10 BamHI-Kartusche der Chloramphenicol-Acetyltransferase (CAT).

Chloramphenicol-Acetyltransferase

met                    stop

GGATCCGAGATTTTCAGGAGCTAAGGAAGCTAAAATG-218 AA-codons-TAA-75bp-GCAGAAATTCGGATCC

BamHI            S/D1        S/D2                                                          BamHI

## FIG. 11 Konstruktion des Plasmids pSAC4 aus dem pSA677 und dem pCM4.